# EUROPÄISCHE PATENTSCHRIFT

(11) **EP 0 895 990 B1**
(45) Veröffentlichungstag und Bekanntmachung des Hinweises auf die Patenterteilung: **03.09.2003**
(21) Anmeldenummer: 98114041.1
(22) Anmeldetag: 28.07.1998
(51) Int. Cl.: C07D 207/16

(54) **Verfahren zur Herstellung von 1-Pyruvyl-L-Prolin**
Method for the preparation of 1-pyruvyl-L-proline
Procédé de préparation de 1-pyruvyl-L-proline

(30) Priorität: 06.08.1997 AT 131997; 12.11.1997 AT 191097
(43) Veröffentlichungstag der Anmeldung: 10.02.1999
(73) Patentinhaber: DSM Fine Chemicals Austria Nfg GmbH & Co KG, 4021 Linz (AT)
(72) Erfinder: Giselbrecht, Karlheinz, Dr., 4061 Pasching (AT); Zimmermann, Curt, Dr., 4310 Mauthausen (AT)
(74) Vertreter: Lindinger, Ingrid

(56) Entgegenhaltungen:
- WO-A-91/04985
- US-A- 5 424 454
- JOU G ET AL: "Total Synthesis of Dehydrodidemnin B. Use of Uronium and Phosphonium Salt Coupling Reagents in Peptide Synthesis in Solution" J. ORG. CHEM. (JOCEAH,00223263);97; VOL.62 (2); PP.354-366, XP002085296 University of Barcelona;Department of Organic Chemistry; Barcelona; E-08028; Spain (ES)

## Beschreibung

1-Pyruvyl-L-Prolin (1-(1,2-Dioxopropyl)-L-prolin; 76391-12-3) stellt ein wertvolles Zwischenprodukt in der Synthese von Dipetiden bzw. Polypeptiden dar, die beispielsweise zu der Klasse der N-Carboxylalkyldipeptid-Inhibitoren von ACE (angiotensin-converting enzyme) gehören.

In der Literatur sind deshalb schon mehrere Varianten zur Herstellung von 1-Pyruvyl-L-Prolin beschrieben. So wird 1-Pyruvyl-L-Prolin (Pyr-Pro-OH) beispielsweise gemäß Jou.G. et al, J. Org. Chem., Vol. 62, No. 2, 1997 p. 354-366 durch Reaktion von L-Prolinbenzylester-Hydrochlorid mit Brenztraubensäure in Gegenwart von Diethanolamin (DIEA), 1-Hydroxybenzotriazol (HOBT) und Dicyclohexylcarbodiimid (DCC) mit anschließender Freisetzung der Säure durch Hydrierung mittels Wasserstoff über Pd/C erhalten.

Der Nachteil dieser Reaktion liegt vorallem in der niedrigen Ausbeute von 36 % des im 1. Reaktionsschritt erhaltenen Esters, wodurch die Gesamtausbeute an Pyr-Pro-OH zwangsläufig ebenfalls nicht sehr hoch ist. Weiters muß der Ester vor Freisetzung der Säure noch aus dem Reaktionsgemisch isoliert und gereinigt werden.

Gemäß Krit N.A. et al, Khim, Farm. Zh 25 (1991), 1, p 44-46 (& Pharm. Chem. J. (Engl. Translation) 25 (1991) 7, p. 482-485) wird L-Prolinbenzylester mit Brenztraubensäurechlorid in DMF und SOCl₂ in Gegenwart von Triethylamin umgesetzt und anschließend wiederum die Säure durch Hydrierung mittels Wasserstoff über Pd/C freigesetzt. Auch bei dieser Variante wird der als Zwischenprodukt gebildete Ester trotz 100 % Überschuß an Säurechlorid in einer schlechten Ausbeute von 40 % erhalten, sodaß die Gesamtausbeute bei diesem Verfahren ebenfalls nicht sehr hoch ist. Weiters muß auch hier der Ester vor dem Freisetzen der Säure zuerst isoliert und gereinigt werden.

Aufgabe der vorliegenden Erfindung war es demnach, ein Verfahren zur Herstellung von Pyr-Pro-OH zu finden, daß ohne Isolierung und Reinigung des gebildeten Zwischenproduktes eine hohe Gesamtausbeute an Pyr-Pro-OH gewährleistet.

Unerwarteterweise konnte diese Aufgabe durch eine Eintopfreaktion mit anschließender Extraktion des Endproduktes gelöst werden.

Gegenstand der vorliegenden Erfindung ist demnach ein Verfahren zur Herstellung von 1-Pyruvyl-L-Prolin, das dadurch gekennzeichnet ist, daß L-Prolin mit Brenztraubensäuremethylester-dimethylketal in Gegenwart eines Alkalimetallalkoxides in einem organischen Lösungsmittel umgesetzt und anschließend eine saure Hydrolyse des gebildeten Ketals durchgeführt wird, worauf 1-Pyruvyl-L-Prolin durch Extraktion mit einem organischen Lösungsmittel aus dem Reaktionsgemisch isoliert wird.

Bei dem erfindungsgemäßen Verfahren wird zuerst L-Prolin mit Brenztraubensäuremethylester-dimethylketal in Gegenwart eines Alkalimetallalkoxides reagieren gelassen. Das als Ausgangsverbindung dienende Dimethylketal ist leicht im großtechnischen Ausmaß und in hohen Ausbeuten herstellbar, beispielsweise durch Umsetzung von Brenztraubensäuremethylester, welcher kommerziell in hoher Reinheit erhältlich ist, mit Methanol unter saurer Katalyse (H₂SO₄, HCI, p-TsOH, H₃PO₄, saure lonenaustauscher u.s.w.) und Entfernen des Reaktionswassers (z.B. mit Orthoameisensäureester) auf chemischem Weg oder destillativ unter Rückführung des Methanols.

Die beiden Ausgangsverbindungen werden dabei bevorzugt äquimolar eingesetzt.

Ein leichter Überschuß des Dimethylketals kann jedoch auch verwendet werden.

Als Alkalimetallalkoxid eignen sich dabei Natrium-, Kalium- oder Lithium- C₁-C₁₀ Alkoxide. C₁-C₁₀ Alkoxide sind beispielsweise Meth-, Eth- oder tert. Butoxid u.s.w.. Bevorzugt wird Natriummethoxid verwendet. Natriummethoxid kann als Feststoff oder als käuflich erhältliche 30 %ige methanolische Lösung eingesetzt werden. Die Reaktion findet in Gegenwart von 1,5 bis 3 Moläquivalenten an Alkalimetallalkoxid statt. Bevorzugt werden 1,8 bis 2,5, besonders bevorzugt 2 Moläquivalente an Alkalimetallalkoxid zugesetzt. Als organisches Lösungsmittel können beispielsweise C₁-C₄-Alkohole wie etwa Methanol, Ethanol, i-Propanol, Ester wie etwa Methylacetat, Ethylacetat u.s.w., Toluol, Ether wie etwa Dioxan, THF oder Methyl-tert.butyl-ether (MtBE) u.s.w. verwendet werden. Bevorzugt werden Dioxan, THF oder Methanol eingesetzt. Gegebenenfalls ist die Zugabe eines Lösungsvermittlers erforderlich, beispielsweise Methanol bei Verwendung von MtBE.

Die Reaktionstemperatur liegt bei der vom eingesetzten Lösungsmittel abhängigen Rückflußtemperatur des Reaktionsgemisches.

Das Reaktionsgemisch wird bis zum Ende der Reaktion bei Rückflußtemperatur gerührt, das Ende der Reaktion wird dabei beispielsweise gaschromatographisch kontrolliert. Bevorzugt wird das Reaktionsgemisch nach beendeter Reaktion noch etwas nachgerührt.

Anschließend wird dem Reaktionsgemisch Wasser zugesetzt, worauf es bei Verwendung eines mit Wasser nicht mischbaren Lösungsmittels zur Phasentrennung kommt. Liegen zwei Phasen vor, wird die organische Phase verworfen und nur die wäßrige Phase weiterverwendet. Die nachfolgende saure Hydrolyse erfolgt bei Temperaturen von 15 bis etwa 50 °C, bevorzugt bei Raumtemperatur.

Durch Zugabe einer anorganischen oder organischen Säure wird ein pH Wert zwischen 1 und 4, bevorzugt zwischen 1 und 2, eingestellt. Als anorganische Säuren eignen sich beispielsweise H₂SO₄, H₃PO₄, HCI, u.s.w. Geeignete organische Säure sind beispielsweise p-Toluolsulfonsäure, Ameisensäure, Essigsäure u.s.w. Bevorzugt werden anorganische Säuren, besonders bevorzugt wird H₂SO₄ eingesetzt.

Das Reaktionsgemisch wird etwa 0,5 bis 15 Stunden, bevorzugt 1 bis 3 Stunden, zur Vervollständigung der Ketalspaltung gerührt.

Ein gegebenenfalls vorhandener Feststoff wird vor der anschließenden Isolierung von Pyr-Pro-OH abgetrennt, beispielsweise durch Abnutschen, Filtrieren u.s.w.

Zur Isolierung von Pyr-Pro-OH wird das Reaktionsgemisch bevorzugt eingeengt und anschließend mit einem organischen Lösungsmittel extrahiert. Geeignete Extraktionsmittel sind beispielsweise Ethylacetat, Methyl-tertbutylether (MtBE) Diethylether, Toluol u.s.w. Bevorzugt werden Ethylacetat und MtBE eingesetzt. Gegebenenfalls wird vor der Extraktion ein pH-Wert von etwa 7, beispielsweise durch Zugabe von NaOH eingestellt um, zum Beispiel bei Verwendung eines Überschußes an Dimethylketal, unumgesetztes Edukt aus dem Reaktionsgemisch abzutrennen.

Die Reinheit von Pyr-Pro-OH wird während der Extraktion mittels Dünnschichtchromatographie, Gaschromatographie oder mittels HPLC überprüft. Bei Erreichen einer entsprechenden Reinheit wird sodann das Extraktionsmittel abdestilliert. Pyr-Pro-OH wird nach dem erfindungsgemäßen Verfahren in Ausbeuten von bis zu 90 % in einfacher Weise durch eine Eintropfreaktion mit anschließender Extraktion erhalten.

### Beispiel 1

Ein Gemisch aus 11,5 g (0,1 mol) L-Prolin, 14,8 g (0,1 mol) Brentraubensäuremethylesterdimethylketal, 10,8 g (0,2 mol) Natriummethoxid und 200 ml Dioxan wurde 5 h bei Rückflußtemperatur (97 - 101 °C) gerührt.

Anschließend wurden 100 ml H₂O zugegeben und mit H₂SO₄ (1:1) ein pH Wert von 1,0 eingestellt. Das Reaktionsgemisch wurde noch etwa 3 h bei Raumtemperatur gerührt und dann der angefallene Feststoff abgenutscht. Das Filtrat wurde zur Hälfte eingeengt und mehrmals mit Ethylacetat extrahiert. Nach Abdestillieren von Ethylacetat wurden 16,4 g Rückstand erhalten.

Ausbeute: 16,4 g 1-Pyruvyl-L-Prolin (= 90 % d. Theorie)

### Beispiel 2

Ein Gemisch aus 11,5 g (1 mol) L-Prolin, 14,8 g (1 mol) Brenztraubensäuremethylester-dimethylketal, 10,8 g (0,2 mol) Natriummethoxid und 200 ml Methyl-tert-Butylether wurde auf Rückflußtemperatur erhitzt (57 °C). Um den verbleibenden Feststoff zu lösen wurden 5 ml Methanol zugegeben und 3 h bei Rückflußtemperatur gerührt. Anschließend wurden 100 g H₂O zugegeben, worauf sich der restliche Feststoff löste und sich 2 Phasen bildeten. Mit H₂SO₄ (1:1) wurde sodann ein pH-Wert von 5,8 eingestellt, die organische Phase verworfen und die wäßrige Phase mit H₂SO₄ (1:1) auf einen pH-Wert von 1,0 gestellt.

Die wäßrige Phase wurde noch 3 h bei 50 °C gerührt, von 150 ml auf 80 ml eingeengt und mit 2N NaOH auf pH 7,0 gestellt.

Nach 2maliger Extraktion mit Ethylacetat wurde wiederum mit H₂SO₄ (1:1) ein pH Wert von 1,3 eingestellt und noch 3 mal mit je 200 ml Ethylacetat extrahiert, das anschließend im Vakuum abdestilliert wurde.

Ausbeute 11,0 g 1-Pyruvyl-L-Prolin (gelbes Öl, ca. 60 % d. Theorie)

## Patentansprüche

1. Verfahren zur Herstellung von N-Pyruvyl-L-Prolin, **dadurch gekennzeichnet, daß** L-Prolin mit Brenztraubensäuremethylester-dimethylketal in Gegenwart eines Alkalimetallalkoxides in einem organischen Lösungsmittel umgesetzt und anschließend eine saure Hydrolyse des gebildeten Ketals durchgeführt wird, worauf 1-Pyruvyl-l-Prolin durch Extraktion mit einem organischen Lösungsmittel aus dem Reaktionsgemisch isoliert wird.

2. Verfahren nach Anspruch 1, **dadurch gekennzeichnet, daß** als Alkalimetallalkoxid Natrium-, Kalium- oder Lithium-C₁-C₁₀ Alkoxide eingesetzt werden.

3. Verfahren nach Anspruch 1, **dadurch gekennzeichnet, daß** als Alkalimetallalkoxid Natriummethoxid eingesetzt wird.

4. Verfahren nach Anspruch 1, **dadurch gekennzeichnet, daß** 1,5 bis 3 Moläquivalente an Alkalimetallalkoxid zugesetzt werden.

5. Verfahren nach Anspruch 1, **dadurch gekennzeichnet, daß** L-Prolin und Brenztraubensäuremethylester-dimethylketal äquimolar eingesetzt werden.

6. Verfahren nach Anspruch 1, **dadurch gekennzeichnet, daß** die Reaktion von L-Prolin und Brenztraubensäuremethylester-dimethylketal bei der Rückflußtemperatur des Reaktionsgemisches durchgeführt wird.

7. Verfahren nach Anspruch 1, **dadurch gekennzeichnet, daß** die saure Hydrolyse bei 15 bis 50 °C durchgeführt wird.

8. Verfahren nach Anspruch 1, **dadurch gekennzeichnet, daß** bei der sauren Hydrolyse ein pH Wert zwischen 1 und 4 mittels Zugabe einer anorganischen oder organischen Säure eingestellt wird.

## Claims

1. Process for preparing N-pyruvyl-L-proline, **characterized in that** it comprises reacting L-proline with methyl pyruvate dimethyl ketal in the presence of an alkali metal alkoxide in an organic solvent and subsequently carrying out an acid hydrolysis of the ketal formed, and isolating 1-pyruvyl-L-proline from the reaction mixture by extraction with an organic solvent.

2. Process according to Claim 1, **characterized in that** the alkali metal alkoxide used is sodium C₁-C₁₀-alkoxide, potassium C₁-C₁₀-alkoxide or lithium C₁-C₁₀-alkoxide.

3. Process according to Claim 1, **characterized in that** the alkali metal alkoxide used is sodium methoxide.

4. Process according to Claim 1, **characterized in that** from 1.5 to 3 mol equivalents of alkali metal alkoxide are added.

5. Process according to Claim 1, **characterized in that** equimolar amounts of L-proline and methyl pyruvate dimethyl ketal are employed.

6. Process according to Claim 1, **characterized in that** the reaction of L-proline and methyl pyruvate dimethyl ketal is carried out at the reflux temperature of the reaction mixture.

7. Process according to Claim 1, **characterized in that** the acid hydrolysis is carried out at from 15 to 50°C.

8. Process according to Claim 1, **characterized in that** a pH between 1 and 4 is set for the acid hydrolysis by addition of an inorganic or organic acid.

## Revendications

1. Procédé de préparation de N-pyruvyl-L-proline, **caractérisé en ce qu'**on fait réagir de la L-proline avec de l'acétone issue de l'ester méthylique de l'acide pyruvique en présence d'un alcoxyde de métal alcalin dans un solvant organique et **en ce qu'**on conduit une hydrolyse acide du cétal formé, la 1-pyruvyl-L proline étant isolée du mélange réactionnel par extraction au moyen d'un solvant organique.

2. Procédé suivant la revendication 1, **caractérisé en ce qu'**on utilise, à titre d'alcoxyde de métal alcalin, des alcoxydes en C₁-C₁₀ de sodium, de potassium ou de lithium.

3. Procédé suivant la revendication 1, **caractérisé en ce qu'**on utilise, à titre d'alcoxyde de métal alcalin, du méthoxyde de sodium.

4. Procédé suivant la revendication 1, **caractérisé en ce qu'**on utilise entre 1,5 et 3 équivalents molaires d'alcoxyde de métal alcalin.

5. Procédé suivant la revendication 1, **caractérisé en ce qu'**on utilise la L-proline et l'acétone issue de l'ester méthylique de l'acide pyruvique en quantités équimolaires.

6. Procédé suivant la revendication 1, **caractérisé en ce qu'**on conduit la réaction entre la L-proline et l'acétone issue de l'ester méthylique de l'acide pyruvique à la température de reflux du mélange réactionnel.

7. Procédé suivant la revendication 1, **caractérisé en ce qu'**on conduit l'hydrolyse acide entre 15 et 50 °C.

8. Procédé suivant la revendication 1, **caractérisé en ce qu'**on ajuste le pH entre 1 et 4 durant l'hydrolyse acide par addition d'un acide inorganique ou organique.
